# EUROPÄISCHE PATENTSCHRIFT

(11) **EP 0 486 806 B1**
(45) Veröffentlichungstag und Bekanntmachung des Hinweises auf die Patenterteilung: **17.01.1996**
(21) Anmeldenummer: 91117335.9
(22) Anmeldetag: 11.10.1991
(51) Int. Cl.: C07D 405/06

(54) **Verfahren zur Herstellung von racemischen Pilosinin-Derivaten**
Process for the preparation of racemic pilosinine derivatives
Procédé pour la préparation des dérivés racémiques de pilosinine

(30) Priorität: 23.10.1990 DE 4033612
(43) Veröffentlichungstag der Anmeldung: 27.05.1992
(73) Patentinhaber: MERCK PATENT GmbH, D-64271 Darmstadt (DE)
(72) Erfinder: Heywang, Ulrich, Dr., W-6100 Darmstadt (DE); Casutt, Michael, Dr., W-6101 Erzhausen (DE); Schwarz, Michael, Dr., W-6080 Gross-Gerau (DE)

(56) Entgegenhaltungen:
- EP-A- 0 199 206
- EP-A- 0 336 250
- EP-A- 0 404 175
- HELVETICA CHIMICA ACTA Bd. 55, Nr. 4, 1. Juni 1972, BASEL, CH Seiten 1053-1062; H. LINK ET AL.: 'Über die Synthese der Pilocarpus-Alkaloide Isopilosin und Pilocarpin, sowie die absolute Konfiguration des (+)-Isopilosins'
- CHEMICAL ABSTRACTS, vol. 90, no. 19, 7. Mai 1979, Columbus, Ohio, US; abstract no. 152419Q, H.W. VOIGTLAENDER ET AL.: 'Epiisopiloturine, a new pilocarpus alkaloid' Seite 638; & Arch. Pharm. (Weinheim, DE) 1978, 311(11), 927-35
- CHEMICAL ABSTRACTS, vol. 81, no. 15, 14. Oktober 1974, Columbus, Ohio, US; abstract no. 91774S, E. TEDESCHI ET AL.: 'Isolation characterization, and synthesis of trans-pilosine stereoisomers occuring in nature. Circular dichroism and mass spectral studies' Seite 488; & J. Org. chem. 1974, 39(13), 1864-70

## Beschreibung

Die Erfindung betrifft ein Verfahren zur Herstellung von racemischen Pilosinin-Derivaten aus einem 5-Formyl-1-alkyl-imidazol der Formel I, 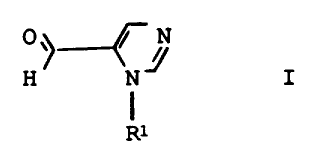 worin
- R¹: eine niedermolekulare geradkettige oder verzweigte Alkylkette mit 1-6 C-Atomen bedeutet.

Besonderes Interesse hat das Derivat mit R¹ = Methyl, das eine Vorstufe von Pilocarpin darstellt und in dieses auf bekanntem Weg überführt werden kann. Andere Pilosininderivate stellen wertvolle Zwischenprodukte zur Herstellung anderer Zielverbindungen dar.

Pilocarpin, ein Imidazol-Alkaloid, ist auf Grund seiner vielseitigen pharmakologischen Eigenschaften Gegenstand zahlreicher Untersuchungen. Seine herausragenden pharmakologischen Wirkungen sind diaphoretische Effekte, Stimulierung des parasympatischen Systems, miotische Wirkungen und insbesondere Anwendungen in der Ophthalmologie.

Verfahren zur Herstellung von racemischem und optisch aktivem Pilocarpin sind bekannt. Alle diese Verfahren sind jedoch durch eine vergleichsweise große Zahl von Syntheseschritten gekennzeichnet, wo nur geringe Gesamtausbeuten erzielt werden.

Bei der Synthese nach H. Link und K. Bernauer (Helv. Chem. Acta 55, 1053 (1972)) dient racemisches Pilosinin als Vorstufe von Pilocarpin. Als Ausgangsmaterial wurde das 5-Formyl-1-methyl-imidazol eingesetzt, das in wenigen Schritten leicht aus Sarcosin zugänglich ist. Das Verfahren von Link und Bernauer beinhaltet die Stobbe-Kondensation eines Imidazolderivats mit Bernsteinsäurediester zu einem Maleinhalbester, dessen regioselektive Reduktion zur Butenolid-Bildung führt, aus dem durch katalytische Reduktion im letzten Schritt das Pilosinin hervorgeht.

Nachteil dieses Verfahrens ist der schlechte Verlauf der Stobbe-Kondensation, wo Ausbeuten von maximal 20 % erzielt werden.

Aufgabe der vorliegenden Erfindung ist es ein Verfahren zur Herstellung von Pilosininderivaten für die Pilocarpin-Synthese aus leicht zugänglichen Ausgangsmaterialien zu finden, das mit hohen Ausbeuten und weitgehend ohne Nebenprodukte verläuft.

Diese Aufgabe wurde überraschend durch das erfindungsgemäße Verfahren gelöst durch die Einführung einer Aldehyd-Schutzgruppe beim 5-Formyl-1-alkyl-imidazol und nachfolgender γ-Lactenon-Addition.

Gegenstand der Erfindung ist daher ein Verfahren zur Herstellung der Pilocarpin-Vorstufe Pilosinin, dadurch gekennzeichnet, daß man
a) 5-Formyl-1-alkyl-imidazole der Formel I, 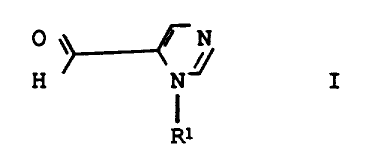 worin
   - R¹: eine niedermolekulare geradkettige oder verzweigte Alkylkette mit 1-6 C-Atomen bedeutet,
   in ein Thioacetal der Formel II überführt 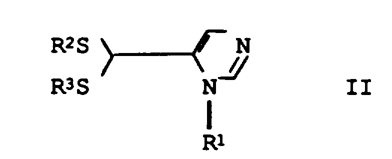 worin
   - R¹: die angegebene Bedeutung besitzt,
   - R und R³: jeweils gleich oder unabhängig voneinander eine niedermolekulare Alkylgruppe mit 1-5 C-Atomen, substituiertes oder unsubstituiertes Phenyl oder Benzyl oder gemeinsam eine Alkylengruppe mit 1-5 C-Atomen bedeutet,
b) das so erhaltene Thioacetal der Formel II in Gegenwart einer Base deprotoniert und mit γ-Crotonlacton zu einer Verbindung der Formel III umsetzt, 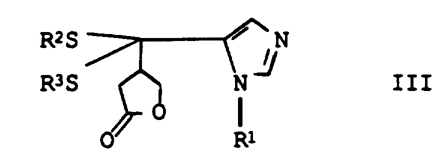 worin
   R¹, R und R³ die angegebene Bedeutung besitzen,
c) die so erhaltene Verbindung III zu Pilosininderivaten der Formel IV reduziert, 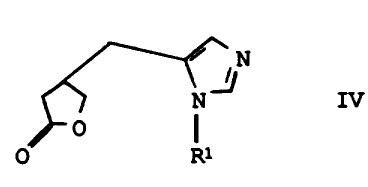 worin
   R¹ die angegebene Bedeutung besitzt.

Die Verbindungen der allgemeinen Formeln II und III sind neu und damit ebenfalls Gegenstand der vorliegenden Erfindung.

Falls R¹, R und R³ einen Alkylrest bedeuten, so kann dieser geradkettig oder verzweigt sein. Verzweigte Alkylreste enthalten in der Regel nicht mehr als eine Kettenverzweigung. Bevorzugte verzweigte Alkylreste sind Isopropyl, 2-Butyl (= 1-Methylpropyl), Isobutyl (= 2-Methylpropyl), 2-Methylbutyl, Isopentyl (= 3-Methylbutyl), 2-Methylpentyl oder 3-Methylpentyl.

Vorzugsweise sind R und R³ geradkettig, besitzen 1, 2, 3, 4 oder 5 C-Atome und bedeuten demnach vorzugsweise Methyl, Ethyl, Propyl, Butyl, Pentyl oder Hexyl.

R¹ bedeutet vorzugsweise Methyl oder Ethyl.

Falls R und R³ einen Phenyl- oder Benzylrest bedeuten, so kann dieser durch Fluor, Chlor, Brom, Alkyl oder Alkoxy mit 1-3 C-Atomen substituiert sein. Im Fall einer Monosubstitution befindet sich der Substituent vorzugsweise in der o- oder p-Position zum Schwefel.

Ausgehend vom 5-Formyl-1-alkyl-imidazol, das leicht aus den entsprechenden Alkyl-imidazol-5-carbonsäuremethylestern durc Lithiumaluminiumhydridreduktion und nachfolgende Oxidation mit aktivem Braunstein gut zugänglich ist (EP 0336 250), erfolgt zunächst die Einführung einer Schutzgruppe für die Aldehydfunktion.

Geeignete Schutzgruppen sind solche, die gegenüber starken Basen inert sind, aber leicht und selektiv durch komplexe Reduktionsmittel abspaltbar sind. Die Einführung der Schutzgruppe erfolgt wie aus der Literatur hinreichend bekannt durch Umsetzung des 5-Formyl-1-alkyl-imidazols der Formel I mit substituierten bzw. unsubstituierten Thiophenolen, Alkanthiolen oder Alkenenthiolen unter Rückfluß. Vorzugsweise erfolgt die Umsetzung der Reaktionspartner in einem geeigneten Lösungsmittel unter Zusatz von mehr als einem Äquivalent p-Toluolsulfonsäure. Geeignete Lösungsmittel sind z.B. aromatische Kohlenwasserstoffe wie Benzol, Xylol und Toluol.

Die Deprotonierung des Thioacetals im folgenden Schritt erfolgt in Gegenwart einer Base. Geeignet sind Alkalimetallorganyle wie Methyllithium, n-Butyllithium, t-Butyllithium und Phenyllithium sowie Amide wie Natriumamid und Lithiumdiisopropylamid. Bei den eingesetzten Lösungsmitteln handelt es sich vorzugsweise um Kohlenwasserstoffe wie Pentan, Hexan, Cyclohexan oder um Ether wie Tetrahydrofuran, Dioxan, tert.-Butylmethylether, Diethylether oder Gemische aus den geeigneten Lösungsmitteln. Die Reaktionstemperaturen liegen zweckmäßig entsprechend der Reaktivität der eingesetzten Base zwischen -90° und -50 °C. Die Michael-Addition an das γ-Lactenon erfolgt in situ im selben Lösungsmittel oder Lösungsmittelgemisch wie die Deprotonierungsreaktion.

Die Methoden zur Abspaltung der Schutzgruppe im nachfolgenden Syntheseschritt sind abhängig von der Art der Reste R und R³.

Vorzugsweise werden komplexe Metallhydride eingesetzt wie Lithiumaluminiumhydrid, Natriumborhydrid, Raney-Nickel oder Stannylhydride, insbesondere Tributylzinnhydrid, in Gegenwart eines Radikalstarters wie Azoisobutyronitril. Als Lösungsmittel kommen je nach Natur des Reduktionsmittels Wasser, Alkohole wie Methanol, Ethanol, Isopropanol, t-Butanol und n-Butanol, Ether wie Tetrahydrofuran, Dioxan und Diethylether sowie Kohlenwasserstoffe wie Pentan, Cyclohexan, Hexan, Benzol, Toluol und Xylol oder Gemische aus den genannten Lösungsmitteln in Betracht. Die Reaktionstemperatur ist sowohl abhängig vom eingesetzten Metallhydrid als auch vom Lösungsmittel. Die Reaktionen erfolgen aber zumeist im Siedebereich des Lösungsmittels.

Die Überführung der Pilosininderivate der Formel IV in Pilocarpinderivate erfolgt auf bekannten Weg zumeist durch Behandlung des Pilosininderivats mit Essigsäureethylester und Kalium-t-butylat. Nach der Hydrierung zu den Alkoholen sowie deren Überführung in die Acetate folgt die Pyrolyse zu den Olefinen, die im letzten Schritt am Platinkontakt hydriert werden.

Das erfindungsgemäße Verfahren erlaubt somit die Herstellung von Pilocarpin und seinen Derivaten aus Pilosininderivaten auf einfache Weise und in hohen Ausbeuten aus leicht zugänglichen Ausgangsstoffen in wenigen zum Teil in situ durchführbaren Syntheseschritten und stellt damit einen wesentlichen Fortschritt auf dem Gebiet der Pilocarpin-Synthese dar.

Die nachfolgenden Beispiele sollen das erfindungsgemäße Verfahren erläutern, ohne es zu begrenzen.

### Beispiele:

### Beispiel 1:

### 5-Dithiophenylmethyl-1-methyl-imidazol

33,0 g (300 mmol) 5-Formyl-1-methyl-imidazol werden in 480 ml Toluol gelöst und mit 68,5 g (360 mmol) 4-Toluolsulfonsäuremonohydrat versetzt. Nach Zugabe von 66,1 g (600 mmol) Thiophenol wird die so entstandene Suspension am Wasserabscheider unter Rückfluß gekocht. Das so erhaltene Zweiphasengemisch wird mehrmals mit 4N Natronlauge gewaschen. Die organische Phase wird über Natriumsulfat getrocknet und anschließend im Vakuum eingeengt. Als Rückstand erhält man 86,8 g (278 mmol, 93 %) 5-Dithiophenylmethyl-1-methyl-imidazol als leicht gelbliches Pulver vom Schmelzpunkt von 78,5 - 80 °C.

### Beispiel 2:

### 5-(1',3'-Dithian-2'-yl)-1-methyl-imidazol

8,3 g (75,4 mmol) 5-Formyl-1-methyl-imidazol werden in 120 ml Toluol gelöst und mit 17,1 g (89,9 mmol) 4-Toluolsulfonsäure-monohydrat versetzt. Nach Zugabe von 8,12 g (75,0 mmol) 1,3-Propandithiol wird die so erhaltene Suspension am Wasserabscheider unter Rückfluß gekocht. Anschließend wird mit 4 N Natronlauge gewaschen, die organische Phase über Natriumsulfat getrocknet und im Vakuum bis zur beginnenden Kristallisation eingeengt. Nach Kühlschranklagerung über Nacht erhält man 9,6 g (47,9 mmol), 64 %) 5- (1',3'-Dithian-2'-yl)-1-methyl-imidazol als weißen Feststoff vom Schmelzpunkt 111,5 - 112,5 °C.

### Beispiel 3:

### 3-(1'-Methyl-imidazol-5' yl-dithiophenylmethyl)-butyrolacton

16,4 g (52,5 mmol) 5-Dithiophenylmethyl-1-methyl-imidazol werden in 240 ml Tetrahydrofuran gelöst und auf -78 °C abgekühlt. Dazu tropft man 52,5 mmol n-BuLi in 32 ml Hexan. Nach 30 Minuten bei -78 °C werden 4,9 ml (69,8 mmol) γ-Crotonlacton zugegeben und nach weiteren 1,5 Stunden werden unter Trockeneiskühlung 250 ml Wasser zugetropft, und man läßt auf Raumtemperatur erwärmen. Anschließend wird mit Essigester gewaschen, mit Eisessig angesäuert und erneut mit Essigester extrahiert. Die organische Phase wird über Magnesiumsulfat getrocknet und im Vakuum eingeengt. Der so erhaltene Rückstand (13,8 g) wird an Kieselgel mit Dichlormethan/Methanol (9 : 1) chromatographiert. Man erhält 6,6 g (16,6 mmol, 32 %) 3-(1'Methyl-imidazol-5'yl-dithiophenylmethyl)-butyrolacton als weißes Pulver vom Schmelzpunkt 80 °C.

### Beispiel 4:

### 3-(2'(1"-Methyl-imidazol-5"yl)-1'3'-dithian-2'yl)-butyrolacton

6,9 g (40 mmol) 5-(1',3'Dithian-2'-yl)-1-methyl-imidazol werden in 190 ml Tetrahydrofuran gelöst und auf -78 °C abgekühlt. Dazu tropft man 40 mmol n-BuLi in 24 ml Hexan und nach 30 Minuten wird bei -78 °C mit 3,8 ml (54 mmol) γ-Crotonlacton versetzt. Nach weiteren 1,5 Stunden bei -78 °C werden 190 ml Wasser zugetropft, und der Ansatz wird auf Raumtemperatur erwärmt. Anschließend wird mit Eisessig angesäuert, die organische Schicht abgetrennt und die wäßrige Phase mehrmals mit Essigester gewaschen. Die vereinigten organischen Extrakte werden über Magnesiumsulfat getrocknet und anschließend wird im Vakuum eingeengt. Der Rückstand wird an Kieselgel mit Dichlormethan/Methanol (9 : 1) chromatographiert. Man erhält so 3,1 g (10,9 mmol, 27 %) 3- (2'(1"-Methyl-imidazol-5"yl)-1'3'-dithian-2'yl)-butyrolacton als gelbes Öl.

### Beispiel 5:

### Pilosinin

3,5 g (9 mmol) 3-(1'-Methyl-imidazol-5'yl-dithiophenylmethyl)-butyrolacton werden in 500 ml Ethanol aufgenommen und mit 15 g frisch zubereitetem Raney-Nickel versetzt und 3,5 Stunden unter Rückfluß gekocht. Die erhaltene Suspension wird über eine Schicht Kieselgur abgesaugt und das Filtrat im Vakuum eingeengt. Zurück bleiben 0,8 g (4,4 mmol, 49 %) Pilosinin.

## Patentansprüche

1. Verfahren zur Herstellung von racemischen Pilosinin-derivaten, dadurch gekennzeichnet, daß man
a) ein 5-Formyl-1-alkyl-imidazol der Formel I 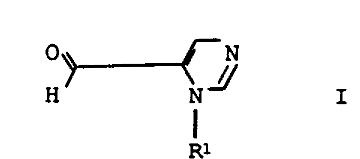 worin
R¹ eine niedermolekulare geradkettige oder verzweigte Alkylkette mit 1-6 C-Atomen bedeutet,
in ein Thioacetal der Formel II überführt, 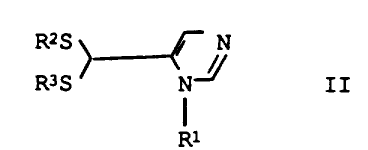 worin
R¹ die angegebene Bedeutung besitzt,
R und R³ jeweils gleich oder unabhängig voneinander eine niedermolekulare Alkylgruppe mit 1-5 C-Atomen, substituiertes oder unsubstituiertes Phenyl oder Benzyl oder gemeinsam eine Alkylengruppe mit 1-5 C-Atomen bedeutet,
b) das so erhaltene Thioacetal der Formel II in Gegenwart einer Base deprotoniert und mit γ-Crotonlacton zu einer Verbindung der Formel III umsetzt, 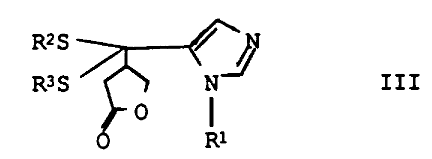 worin
R¹, R und R³ die angegebene Bedeutung besitzen,
c) die so erhaltene Verbindung III zu Pilosininderivaten der Formel IV reduziert, 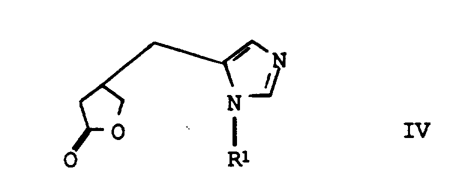 worin
R¹ die angegebene Bedeutung besitzt.

2. Verfahren nach Anspruch 1, dadurch gekennzeichnet, daß man die Verbindung der Formel II mit einem Alkalimetallorganyl als Base deprotoniert.

3. Verfahren nach Anspruch 1, dadurch gekennzeichnet, daß die Reduktion der Verbindung der Formel III zu den Pilosininderivaten mit einem komplexen Metallhydrid oder mit Raney-Nickel erfolgt.

4. Eine Verbindung der allgemeinen Formel II gemäß Anspruch 1.

5. Eine Verbindung der allgemeinen Formel III gemäß Anspruch 1.

## Claims

1. Process for the preparation of racemic pilosinine derivatives, characterised in that
a) a 5-formyl-1-alkylimidazole of the formula I 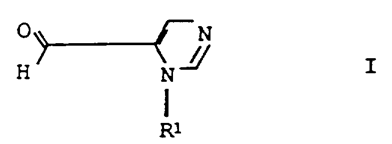 in which
R¹ is a low molecular weight straight-chain or branched alkyl chain with 1-6 C atoms, is converted into a thioacetal of the formula II 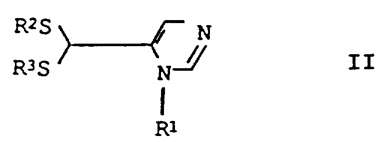
in which
R¹ has the stated meaning,
R and R³ are each, identically or independently of one another, a low molecular weight alkyl group with 1-5 C atoms, substituted or unsubstituted phenyl or benzyl or together are an alkylene group with 1-5 C atoms,
b) the thioacetal of the formula II obtained in this way is deprotonated in the presence of a base and reacted with y-crotonolactone to give a compound of the formula III 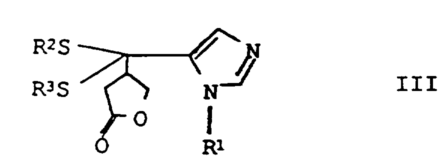 in which
R¹, R and R³ have the stated meaning,
c) the compound III obtained in this way is reduced to pilosinine derivatives of the formula IV 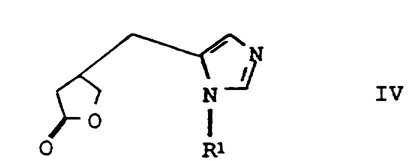 in which
R¹ has the stated meaning.

2. Process according to Claim 1, characterised in that the compound of the formula II is deprotonated with an organic alkali metal compound as base.

3. Process according to Claim 1, characterised in that the reduction of the compound of the formula III to the pilosinine derivatives is carried out with a complex metal hydride or with Raney nickel.

4. A compound of the general formula II according to Claim 1.

5. A compound of the general formula III according to Claim 1.

## Revendications

1. Procédé de préparation de dérivés de la pilosinine à l'état de racémiques, caractérisé en ce que :
a) on convertit un 5-formyl-1-alkylimidazole de formule I : 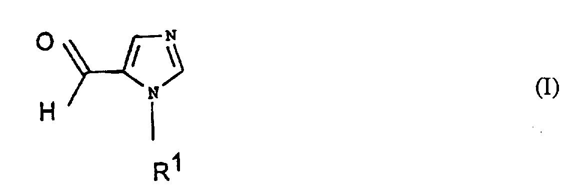 dans laquelle
R¹ représente une chaîne alkyle de faible poids moléculaire linéaire ou ramifiée en C₁-C₆, en un thioacétal de formule II : 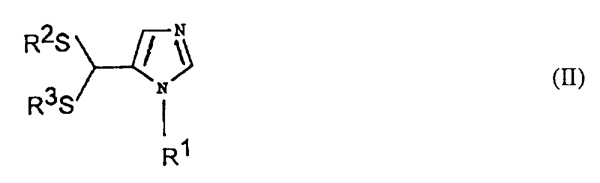
dans laquelle :
R¹ a la signification indiquée ci-dessus,
R et R³, ayant les significations identiques ou différentes, représentent chacun, indépendamment l'un de l'autre, un groupe alkyle de faible poids moléculaire en C₁-C₅, un groupe phényle ou benzyle substitué ou non; ou bien forment ensemble un groupe alkylène en C₁-C₅,
b) on déprotonise le thioacétal de formule II ainsi obtenu en présence d'une base et on le convertit avec la γ-crotonolactone en un composé de formule III : 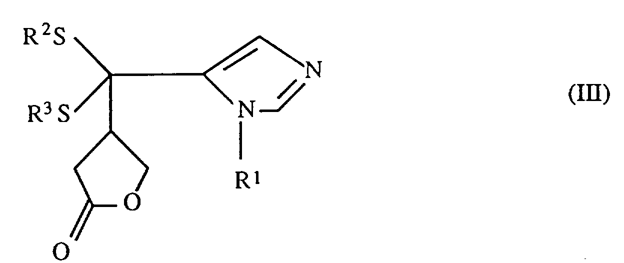 dans laquelle R¹, R et R³ ont les significations indiquées ci-dessus,
c) on réduit le composé III ainsi obtenu en un dérivé de la pilosinine répondant à la formule IV : 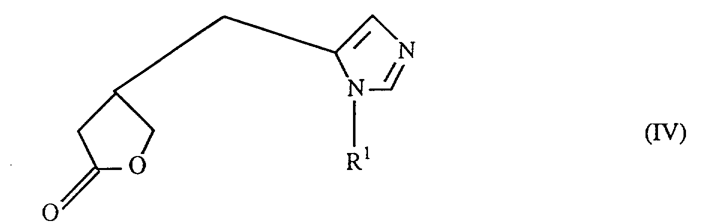 dans laquelle R¹ a la signification indiquée ci-dessus.

2. Procédé selon la revendication 1, caractérisé en ce que l'on déprotonise le composé de formule II à l'aide d'une base consistant en un métal alcalin-organyle.

3. Procédé selon la revendication 1, caractérisé en ce que l'on réduit le composé de formule III en dérivé de la pilosinine à l'aide d'un hydrure métallique complexe ou du nickel de Raney.

4. Un composé de formule générale II selon la revendication 1.

5. Un composé de formule générale III selon la revendication 1.
